# EUROPEAN PATENT APPLICATION

(11) **EP 2 883 873 A1**
(43) Date of publication of application: **17.06.2015**
(21) Application number: 13306735.5
(22) Date of filing: 16.12.2013
(51) Int. Cl.: C07D 319/06, C08G 12/20

(54) **Six-membered cyclic biscarbonates for the preparation of polymers**

(71) Applicant: UNIVERSITE DE BORDEAUX I, 33400 Talence Cedex (FR); Institut Polytechnique de Bordeaux, 33400 Talence (FR); Centre National de la Recherche Scientifique (CNRS), 75016 Paris (FR); Institut des Corps Gras Etudes et Recherches Techniques-Iterg, 33600 Pessac (FR)
(72) Inventor: Cramail, Henri, 33350 SAINTE TERRE (FR); Maisonneuve, Lise, 33400 TALENCE (FR); Grau, Etienne, 33400 TALENCE (FR); Alfos, Carine, 33600 PESSAC (FR)
(74) Representative: Blot, Philippe Robert Emile

(57) **Abstract**

The present invention relates to six-membered cyclic biscarbonates of formula: 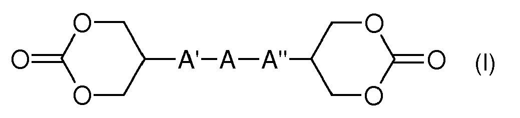 wherein:
- A is selected from the group consisting of -CH=CH- and -CH₂CHR'-S-R-S-CHR"CH₂-, wherein R is a linear or branched alkylene radical in C₂-C₆, optionally interrupted by one or more oxygen atoms, and wherein R' and R", identical or different, are selected from the group consisting of hydrogen atom and linear alkyl radicals in C₁-C₁₀; and
- A' and A", identical or different, are linear or branched alkylene radicals in C₁-C₁₂,
on the proviso that, when A is -CH₂CH₂-S-R-S-CH₂CH₂-, A' and A" are in C₂-C₁₂, and their use for the preparation of polymers, such as poly(hydroxyurethane)s.

## Description

The present invention concerns six-membered cyclic biscarbonates and their use for the preparation of polymers, such as poly(hydroxyurethane)s.

The most explored alternative route to non-phosgene and non-isocyanate polyurethanes is based on the reaction of 5-membered cyclic carbonates and amines. This strategy has been investigated to prepare cross-linked as well as linear poly(hydroxyurethane)s.

However, the reactivity of 5-membered cyclic carbonates needs to be increased.

Indeed, while regarding the literature, few authors have developed poly(hydroxyurethane)s from more reactive cyclic carbonates. For instance, Tomita et al. synthesized 6 and 7-membered cyclic carbonates and their corresponding poly(hydroxyurethane)s and bifunctional compounds (Tomita et al. Journal of Polymer Science Part A: Polymer Chemistry, 2000, 39, 162-168; Tomita et al. Journal of Polymer Science Part A: Polymer Chemistry, 2001, 39, 860-867; Tomita et al. Journal of Polymer Science Part A: Polymer Chemistry, 2001, 39, 4091-4100). Tomita et al. demonstrated that 6-membered cyclic carbonates are more reactive than 5-membered ones.

Thus, there is a need for more reactive carbonate monomers, suitable for the preparation of polymers, such as polyurethanes.

While vegetable oil-based 5-membered cyclic carbonates have been studied as poly(hydroxyurethane)s monomers, no literature has been published on the preparation of vegetable oil-based 6-membered cyclic carbonates.

Thus, there is a need for vegetable oil-based carbonate monomers, suitable for the preparation of non-phosgene and non-isocyanate polymers.

Therefore, the present invention provides 6-membered cyclic bifunctional carbonates, also called biscarbonates 6CC, which are useful for the preparation of non-phosgene and non-isocyanate polyurethanes.

Preferably, the 6-membered cyclic bifunctional carbonates of the invention are obtained from renewable sources, such as fatty acids comprised in vegetable oils.

### Biscarbonates

The present invention relates to a compound of formula (I): 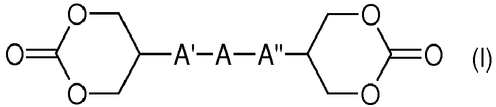 wherein:
- A is selected from the group consisting of -CH=CH- and -CH₂CHR'-S-R-S-CHR"CH₂-, wherein R is a linear or branched alkylene radical in C₂-C₆, preferably in C₄, optionally interrupted by one or more oxygen atoms, and wherein R' and R", identical or different, are selected from the group consisting of hydrogen atom and linear alkyl radicals in C₁-C_{10;} and
- A' and A", identical or different, are linear or branched alkylene radicals in C₁-C₁₂, preferably in C₆-C₁₂,
on the proviso that, when A is -CH₂CH₂-S-R-S-CH₂CH₂-, A' and A" are in C₂-C₁₂.

Within the framework of the present invention, the term "alkyl" means a saturated aliphatic hydrocarbon group, which may be linear or branched, having 1 to 24 carbon atoms in the chain. "Branched" means that one or more alkyl groups such as methyl, ethyl or propyl are attached to a linear alkyl chain.

Within the framework of the present invention, the term "alkylene" means a divalent saturated aliphatic hydrocarbon radical, which may be linear or branched, having 1 to 24 carbon atoms in the chain.

The compounds of the invention are even more reactive than the corresponding 5-membered cyclic carbonates, also called 5CC.

The biscarbonates 6CC of the invention are useful for the preparation of bio-based non isocyanate thermoplastic polyurethanes under milder conditions than those requested for biscarbonates 5CC.

Preferably, in formula (I), A' and A" are in C₆-C₁₂, advantageously in C₇-C₁₂, for example in C₇.

According to one embodiment, the compounds of the invention are obtained from fatty acids and are thus based on renewable sources.

Preferably, in formula (I), R is a linear alkylene radical in C₂-C₆, for example in C₄.

Preferably, in formula (I), A is -CH=CH-.

According to one embodiment, in formula (I), the chain -A'-A-A"- comprises from 4 to 30 carbon atoms, preferably from 12 to 30 carbon atoms, advantageously from 16 to 24 carbon atoms.

According to one embodiment, in formula (I), A' and A" are identical.

According to one embodiment, in formula (I), R' and R" are identical.

Preferably, in formula (I), R' and R" are a hydrogen atom.

According to one variant of the present invention, when A is -CH=CH-, the compounds of the invention are of formula (I-1): 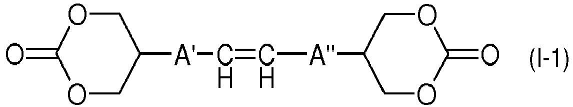 wherein A' and A" are as defined above in formula (I).

According to this variant of the present invention, in formula (I-1), A' and A" are preferably linear alkylene radicals in C₆-C₁₂, preferably in C₇-C₁₂, for example in C₇.

In particular, the present invention relates to the following compound (called UndC20-b6CC in the examples below):

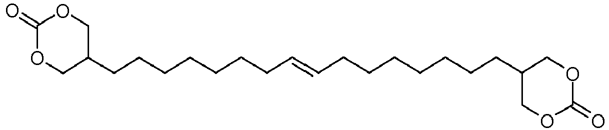

UndC20-b6CC corresponds to a compound of formula (I) wherein A' and A" are -C₇H₁₄- and A is -CH=CH-.

UndC20-b6CC corresponds to a compound of formula (I-1) wherein A' and A" are -C₇H₁₄-.

The present invention also relates to a method for preparing the compound of formula (I-1) as defined above.

A method for preparing the compounds of formula (I-1) as defined above comprises a coupling step by metathesis reaction between a compound of formula (II-1): 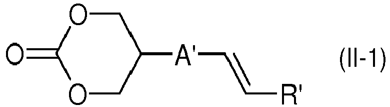 and a compound of formula (II-2): 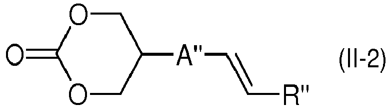 wherein A', A", R' and R" are as defined above in formula (I);
said step being carried out in a solvent in the presence of a metathesis catalyst, preferably selected from the Grubbs' catalysts.

According to one embodiment, in formulae (II-1) and (II-2), A' and A" are linear alkylene radicals in C₆-C₁₂, preferably in C₇-C₁₂, for example in C₇.

According to one embodiment, in formulae (II-1) and (II-2), R' and R" are a hydrogen atom.

The double bonds of the compounds of formulae (II-1) and (II-2) are more reactive when R' = R" = H.

According to one embodiment, in formulae (II-1) and (II-2), A' and A" are identical, and R' and R" are identical.

According to the present invention, a "metathesis catalyst" is a catalyst suitable for coupling a compound of formula (II-1) and a compound of formula (II-2) by metathesis. One may cite the catalysts developed by Grubbs (also referred as Grubbs' catalysts), such as the first generation, the second generation and the third generation Grubbs catalyst.

The coupling step by metathesis reaction is preferably carried out in pentane at room temperature.

According to another variant of the present invention, when A is -CH₂CHR'-S-R-S-CHR"CH₂-, the compounds of the invention are of formula (I-2): 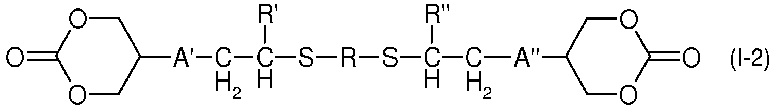 wherein A', A", R, R' and R" are as defined above in formula (I).

According to this other variant of the present invention, in formula (I-2), A is preferably -CH₂CH₂-S-R-S-CH₂CH₂-, R being as defined above in formula (I), and A' and A" are preferably linear alkylene radicals in C₆-C₁₂, preferably in C₇-C₁₂, for example in C₇.

In particular, the present invention relates to the following compound (called UndS-b6CC in the examples below):

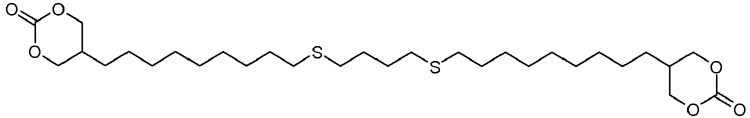

UndS-b6CC corresponds to a compound of formula (I) wherein A' and A" are -C₇H₁₄-and A is -CH₂CH₂-S-CH₂CH₂CH₂CH₂-S-CH₂CH₂-.

UndS-b6CC corresponds to a compound of formula (I-2) wherein A' and A" are -C₇H₁₄-, R is -CH₂CH₂CH₂CH₂-, and R' and R" are H.

The present invention also relates to a method for preparing the compound of formula (I-2) as defined above.

A method for preparing the compounds of formula (I-2) as defined above comprises a coupling step by thiol-ene reaction between a compound of formula (II-1): 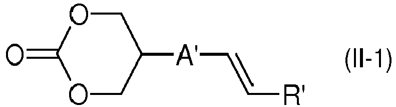 and a compound of formula (II-2): 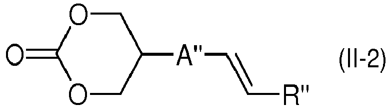 in the presence of a compound of formula HS-R-SH, preferably used as a solvent,
wherein A', A", R, R' and R" are as defined above in formula (I).

According to one embodiment, in formulae (II-1) and (II-2), A' and A" are linear alkylene radicals in C₆-C₁₂, preferably in C₇-C₁₂, for example in C₇.

According to one embodiment, in formulae (II-1) and (II-2), R' and R" are a hydrogen atom.

The double bonds of the compounds of formulae (II-1) and (II-2) are more reactive when R' = R" = H.

According to one embodiment, in formulae (II-1) and (II-2), A' and A" are identical, and R' and R" are identical.

According to the present invention, "used as a solvent" means that, in the coupling step reaction, the compounds of formula (II-1) and (II-2) are in the presence of a large excess of the compound of formula HS-R-SH. Preferably, the reaction mixture consists essentially of the compounds of formula (II-1) and (II-2) and the compound of formula HS-R-SH.

The coupling step by thiol-ene reaction is preferably carried out under UV-irradiation (for example at 264 nm), at room temperature. Alternatively, the reaction may be carried out by heating the mixture of the compounds of formulae (II-1) and (II-2) and the compound of formula HS-R-SH in the presence of a radical initiator, such as AlBN. The reaction may also be carried out under UV-irradiation and heating.

According to one embodiment, the compounds of formula (II-1) and (II-2) are obtained from fatty acids. The method of preparation of the invention enables the preparation of biscarbonates compounds from renewable sources, such as fatty acids comprised in vegetable oils.

According to the present invention, the compound of formula (II-1) and the compound of formula (II-2) may be prepared by a cyclization step of a compound of formula (III): 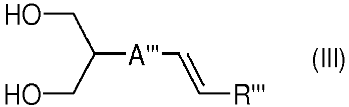 in the presence of a base and a compound of formula X-C(O)-O-R₁, wherein:
- A"' represents either A' or A", A' and A" being as defined above in formula (I), (II-1) and (II-2),
- R"' represents either R' or R", R' and R" being as defined above in formula (I), (II-1) and (II-2),
- X is OR₂, Br or Cl, preferably Cl, and
- R₁ and R₂, identical or different, are linear alkyl radicals in C₁-C₆, preferably in C₁-C₂.

According to one embodiment, in formula (III), R"' is a hydrogen atom.

According to one embodiment, in formulae (III), A"' is a linear alkylene radical in C₆-C₁₂, preferably in C₇-C₁₂, for example in C₇.

Preferably, the base is an organic amine, such as triethylamine.

Preferably, the compound of formula X-C(O)-O-R₁ is ethyl chloroformate.

Preferably, the cyclization step is carried out in a solvent, such as tetrahydrofuran, dioxane or diethyl ether. Preferably, the solvent is tetrahydrofuran.

The cyclization step is generally carried out at room temperature.

According to the present invention, the compound of formula (III) may be prepared by a reduction step of a compound of formula (IV): 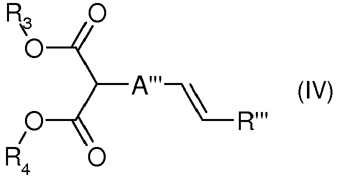 wherein:
- A"' and R"' are as defined above in formula (III), and
- R₃ and R₄, identical or different, are linear alkyl radicals in C₁-C₆, preferably in C₁,
this step being conducted in a solvent in the presence of a metal hydride, such as LiAlH₄, or under pressurized H₂ in the presence of a catalyst, such as palladium over charcoal.

In the above reduction step, when a metal hydride is used, it is generally used in excess in respect of the compound of formula (IV), i.e. in an amount of at least 4 equivalents.

In the above reduction step, when a metal hydride is used, the solvent may be selected form the group consisting of tetrahydrofuran, diethyl ether or dioxane. Preferably, the solvent is tetrahydrofuran.

In the above reduction step, when pressurized H₂ is used, the solvent may be selected form the group consisting of ethanol, methanol, chloroform, tetrahydrofuran or dichloromethane.

The cyclization step is generally carried out at a temperature comprised from 50°C to 100°C, for example at about 80 °C.

According to the present invention, the compound of formula (IV) may be prepared by a reaction step between a compound of formula (V): 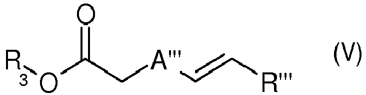 and a compound of formula R₄O-C(O)-OR₄,
wherein:
- A"' and R"' are as defined above in formula (IV), and
- R₃ and R₄ are as defined above in formula (IV),
this step being conducted in the presence of dimethylformamide and a metal hydride, such as NaH.

In the above reaction step, the metal hydride is generally used in excess in respect of the compound of formula (V), i.e. in an amount of at least 4 equivalents.

In the above reaction step, the compound of formula R₄O-C(O)-OR₄ is generally used in large excess in respect of the compound of formula (V), i.e. in an amount of at least 10 equivalents.

Preferably, the compound of formula R₄O-C(O)-OR₄ is dimethyl carbonate.

The above reaction step is generally carried out at a temperature comprised from 40°C to 80°C, for example at about 60°C.

### Poly(hydroxyurethane)s

The present invention also relates to the use of a compound of formula (I) as defined above, for the preparation of poly(hydroxyurethane)s.

The present invention also relates to the polymer susceptible to be obtained by polymerization of a compound of formula (I) as defined above and at least one polyamine.

Said polymer is a poly(hydroxyurethane).

According to one embodiment, the polyamine is a diamine of formula H₂N-B-NH₂, wherein B is chosen from the group consisting of:
- a linear or branched alkylene radical in C₁-C₂₀₀, preferably in C₁-C₃₀, wherein one or more carbon atom(s) is optionally replaced by an oxygen atom;
- an arylene radical, preferably in C₆, optionally functionalized in ortho, meta or para with a linear or branched alkyl group in C₁-C₁₀ wherein one or more carbon atom(s) is optionally replaced by an oxygen atom; and
- a radical of formula -B₁-B₂- wherein:
   o B₁ is a cycloalkylene radical in C₃-C₁₅, wherein one or more carbon atom(s) is optionally substituted by at least one alkyl group in C₁-C₁₅, and
   o B₂ is a linear or branched alkylene radical in C₁-C₁₅.

Within the framework of the present invention, the term "arylene" means a monocyclic, dicyclic, or tricyclic divalent aromatic hydrocarbon radical, having 6 to 14 carbon atoms.

Within the framework of the present invention, the term "cycloalkylene" means a monocyclic, dicyclic, or tricyclic divalent saturated hydrocarbon radical, monocyclic, dicyclic or tricyclic, having 3 to 20 carbon atoms.

The diamine may be:
- 1,4-diaminobutane (4DA): 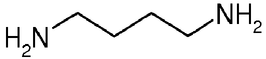
- 1,12-diaminododecane (12DA): 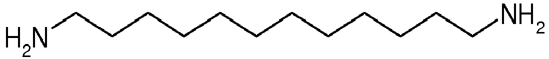
- isophorone diamine (IPDA): 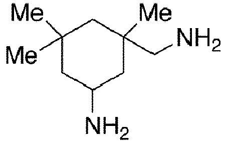
- Priamine 1075 from CRODA,
- the diamines derived from dimerized fatty acids, such as diacids Empol® (from BASF) or Unidyme® from Arizona Chemical, or
- a diamine of formula: 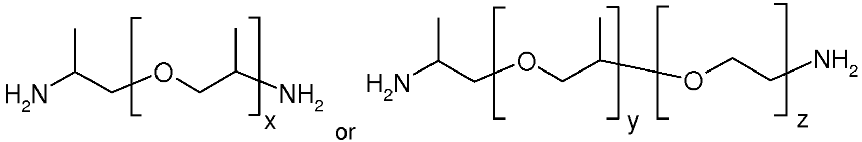 wherein x or y+z being comprised between 2.5 and 68.

According to one embodiment, the polyamine is a diamine of formula H₂N-B-NH₂, wherein B is a linear alkylene radical in C₂-C₂₄, preferably in C₆-C₁₈, for example in C₁₂.

Preferably, the polyamine is 1,12-diaminododecane.

When the polyamine is a diamine, such as 12DA, the poly(hydroxyurethane)s of the invention are thermoplastic linear copolymers.

According to this embodiment, the polymer susceptible to be obtained by polymerization of a compound of formula (I) and a diamine of formula H₂N-B-NH₂ (also called polymer P1) consists in n repetitive units of formula (U-1): 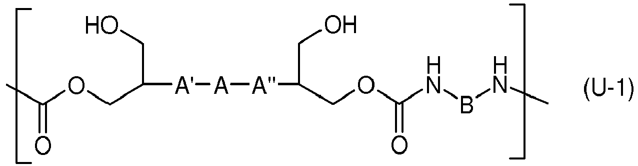 wherein n is comprised from 2 to 200, preferably from 10 to 200, A is as defined above in formula (I), and A' and A" are as defined above in formula (I), (II-1) and (II-2).

According to one embodiment, the polymer P1 may consist in n repetitive units of formula (U-1-1): 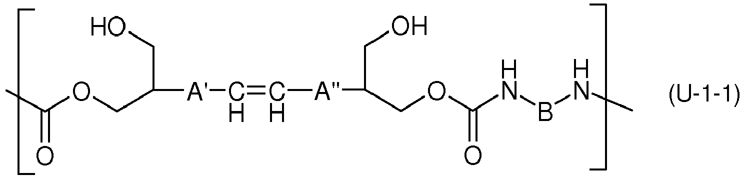 wherein n is comprised from 2 to 200, preferably from 10 to 200, A' and A" are as defined above in formula (U-1).

According to another embodiment, the polymer P1 may consist in n repetitive units of formula (U-1-2): 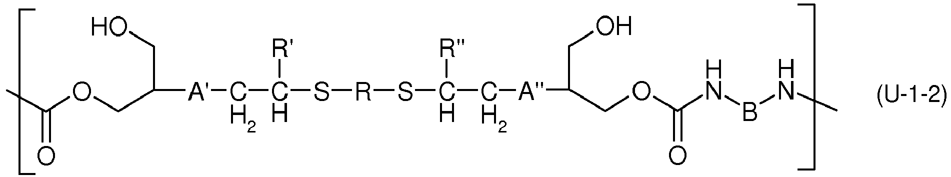 wherein n is comprised from 2 to 200, preferably from 10 to 200, A' and A" are as defined above in formula (U-1), R, R' and R" are as defined in above formula (I).

Preferably, in formulae (U-1), (U-1-1) and (U-1-2), A' and A" are linear alkylene radicals in C₆-C₁₂, preferably in C₇-C₁₂, for example in C₇.

Preferably, in formula (U-1-2), R' and R" are hydrogen atoms.

The present invention also relates to a method for preparing the above-defined polymer P1.

The present invention also relates to a method for preparing the above-defined polymer P1, comprising a step of polymerization of a compound of formula (I) with a diamine of formula H₂N-B-NH₂ as defined above, said step being carried out in bulk or in a solvent and at a temperature comprised from 20°C to 100°C, preferably from 20°C to 60°C.

According to the present invention, "in bulk" means that the polymerization is carried out with a mixture consisting of at least one compound of formula (I) and at least one diamine of formula H₂N-B-NH₂, without any solvent.

The polymerization is preferably carried out in dimethylformamide. The dilution of the reaction mixture comprising the monomers may be of about 1 mol.L⁻¹. The polymerization is carried out at a temperature comprised from 20°C to 100°C, for example either at room temperature or at 50°C.

With the 6-membered cyclic biscarbonates of the invention, poly(hydroxyurethane)s are obtained at a lower temperature compared to the corresponding 5-membered cyclic biscarbonates.

### Polycarbonates

The present invention also relates to the use of the compound of formula (I) as defined above, for the preparation of polycarbonates.

The present invention also relates to the polymer susceptible to be obtained by homopolymerization of a compound of formula (I) as defined above.

### Monocarbonate

The present invention also relates to a compound of formula (II-1): 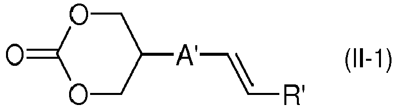 wherein:
- A' is a linear or branched alkylene radical in C₂-C₁₂, preferably in C₆-C₁₂, and
- R' is selected from the group consisting of hydrogen atom and linear alkyl radicals in C₁-C₁₀.

According to one embodiment, in formula (II-1), A' is a linear alkylene in C₇-C₁₂, for example in C₇.

According to one embodiment, in formula (II-1), R' is a hydrogen atom.

The compounds of formula (II-1) are also called monocarbonates.

The present invention also relates to the use of a compound of formula (II-1) as defined above, for the preparation of polycarbonates.

The compounds of formula (II-1) are useful for the preparation of polymers by polymerization. The monocarbonates of formula (II-1) may be obtained from fatty acids. Thus they represent an interesting way to produce polymer from renewable sources.

The present invention also relates to the polymer susceptible to be obtained by homopolymerization of a compound of formula (II-1) as defined above (also called polymer P2).

Said polymer P2 is a linear polycarbonate, preferably a thermoplastic polycarbonate.

Polycarbonates may be obtained by ring-opening polymerization of the compounds of formula (II-1), preferably in the presence of a catalyst such as a base, in bulk or in a solvent, at a temperature comprised from 50°C to 150°C.

According to one embodiment, the polymer P2 susceptible to be obtained by polymerization of a compound of formula (II-1) consists in n repetitive units of formula (U-2): 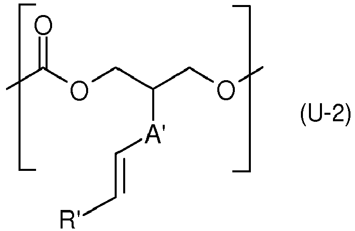 wherein n is comprised from 2 to 200, preferably from 10 to 200, A' and R' are as defined above in formula (II-1).

The present invention also relates to a method for preparing the above-defined polymer P2, comprising a step of polymerization of a compound of formula (II-1) as defined above, said step being carried out in bulk or in a solvent and at a temperature comprised from 30°C to 150°C.

### Examples

### Reactants:

Methyl 10-undecenoate (>96.0%), dodecane-1,12-diamine (12DA, >98%), 1,3-dioxane-2-one (trimethylene carbonate, >98%), and 4-(hydroxymethyl)-1,3-dioxolan-2-one (glycerol carbonate, 5CCOH, >90%) were supplied by TCI, Europe.

Dimethyl carbonate (DMC, 99%) and triethyl amine (TEA, 99%), were purchased from Alfa Aesar.

Sodium hydrate (NaH) (60 % dispersion in mineral oil), N,N-dimethylformamide (DMF, anhydrous grade), lithium aluminum hydride (LiAlH₄) (95%), ethyl chloroformate (97%), 1,4-butanedithiol (>97%), ethylvinyl ether (99%), Grubbs 3^{rd} generation metathesis catalyst, hexylamine (99%), and ethylene carbonate (5CC, 98%) were obtained from Sigma-Aldrich.

Propylene carbonate (5CCMe, 99.5%) was obtained from Fisher.

All products and solvents (reagent grade) were used as received except otherwise mentioned. The solvents were of reagent grade quality and were purified wherever necessary according to the methods reported in the literature.

### Example 1 - Synthesis and characterizations of biscarbonates

### Step 1 - Malonate (Und-Malonate) synthesis

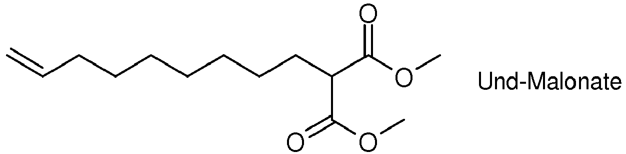

The methyl undecenoate (20 g, 100.9 mmol) was stirred with DMC (340 mL, 4.0 mol, 40 eq), NaH via a 60 wt% dispersion in mineral oil (6 g, 252.1 mmol, 2.5 eq) and DMF (7.8-mL, 109.9 mmol, 1 eq) at 60°C. After 24 hours of reaction, 435 mL of diluted hydrochloric acid was slowly added to the reaction mixture. The organic phase was then washed twice with water, dried over anhydrous sodium sulfate, filtered and then the remaining DMC was removed on rotary evaporator. The compound ***Und-malonate*** was purified by flash chromatography using a mixture of cyclohexane and ethyl acetate and obtained as a viscous liquid. Yield=58%. ¹H NMR (CDCl₃, 25°C, 400 MHz) δ (ppm): 5.79 (m, 1 H), 4.95 (m, 2H), 3.73 and 3.71 (s, 6H), 3.35 (t, 1 H), 2.04 (m, 2H), 1.88 (m, 2H), 1.29 (m, 10H). ¹³C NMR (CDCl₃, 25°C, 100 MHz) δ (ppm): 170.12 (COOCH₃), 139.28 (CH=CH₂), 114.32 (CH=CH₂), 52.57 (C=OOCH₃), 51.87 (CH-(C=OOCH₃)₂), 33.89 (CH₂-CH=CH₂), 29.27-27.46 (CH₂). IR (cm⁻¹): 2924, 2854, 1734.

### Step 2 - Reduction of the malonate: 1, 3-diol (Und-1,3-diol) synthesis

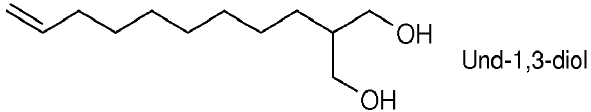

A solution of Und-malonate obtained following Step 1 (10 g, 39.0 mmol) in THF (10 mL) was added to a solution of LiAlH₄ (6.1 g, 160.9 mmol, 4.1 eq.) in THF (80 mL) at 0°C. After the addition was completed, the reaction mixture was allowed to reach slowly room temperature and was refluxed at 80°C for 2 h. The reaction mixture was then cooled to 0°C, and distilled water followed by hydrochloric acid solution (2N) was added dropwise. The product was then extracted three times with ethyl acetate. The organic layer was washed twice with NaCl saturated solution and water, dried over anhydrous sodium sulfate, filtered and then the solvent was removed on rotary evaporator. The ***Und-1,3-diol*** was purified by flash chromatography using a mixture of cyclohexane and ethyl acetate. Yield=66%. ¹H NMR (CDCl₃, 25°C, 400 MHz) δ (ppm): 5.81 (m, 1 H), 4.93 (m, 2H), 3.78-3.63 (m, 4H), 2.96 (s, 2.OH), 2.02 (m, 2H), 1.75 (m, 1 H), 1.36-1.22 (m, 12H). ¹³C NMR (CDCl₃, 25°C, 100 MHz) δ (ppm): 139.42 (CH=CH₂), 114.41 (CH=CH₂), 67.02 (CH-CH₂-OH), 42.18 (CH-CH₂-OH), 34.02 (CH₂-CH=CH₂), 30.05-27.44 (CH₂). IR (cm⁻¹): 3277, 2919, 2850.

### Step 3 - Cyclization: 6-membered cyclic carbonate synthesis (Und-6CC)

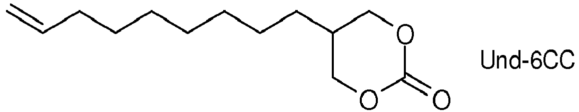

To a solution of triethylamine (10.1 g, 100 mmol, 2 eq.) in THF (400 mL), Und-1,3-diol obtained following Step 2 (10 g, 50 mmol) was added. Then, ethyl chloroformate (10.8 g, 100 mmol) was added to the mixture at 0°C. The reaction mixture was stirred at room temperature for 7 hours. Precipitated triethylamine hydrochloride was filtered off, and the filtrate was concentrated under vacuum. The ***Und-6CC*** was isolated from the reaction mixture by flash chromatography using a mixture of cyclohexane and ethyl acetate and obtained as a viscous liquid with 99.5% purity determined by GC-FID. Yield=75%. ¹H NMR (CDCl₃, 25°C, 400 MHz) δ (ppm): 5.81 (m, 1 H), 4.96 (m, 2H), 4.40 (m, 2H), 4.09 (m, 2H), 2.21 (m, 1 H), 2.05 (m, 2H), 1.35-1.30 (m, 12H). ¹³C NMR (CDCl₃, 25°C, 100 MHz) δ (ppm): 148.72 (OCOO), 139.14 (CH=CH₂), 114.42 (CH=CH₂), 72.24 (CH₂-OCOO), 33.84 (CH₂-CH=CH₂), 31.39 (CH-CH₂-OCOO), 29.53-26.69 (CH₂). IR (cm⁻¹): 2924, 2856, 1753.

### Step 4A - Metathesis reaction: 6-membered cyclic biscarbonate synthesis (UndC20-b6CC)

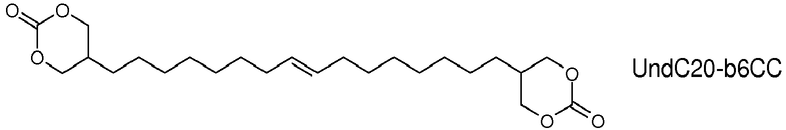

Into a round-bottom flask equipped with a mineral oil bubbler, the Und-6CC obtained following Step 3 (4 g, 17.7 mmol) was charged with dried pentane (40mL) and 3^{rd} generation Grubbs catalyst (78.2 mg, 0.088 mmol). The contents were vigorously stirred at room temperature for 4 hours. Afterwards, 3.8 mL of ethylvinyl ether was added to deactivate the Grubbs catalyst. The equilibrium was drived thank to the precipitation of the formed product. The product was then purified with flash chromatography using a mixture of cyclohexane and ethyl acetate as eluent. ***UndC20-b6CC*** was obtained as a grey solid. Yield=51.5%. ¹H NMR (CDCl₃, 25°C, 400 MHz) δ (ppm): 5.37 (m, 2H), 4.41 (m, 4H), 4.09 (m, 4H), 2.20 (m, 2H), 1.97 (m, 4H), 1.35-1.28 (m, 22H). ¹³C NMR (CDCl₃, 25°C, 100 MHz) δ (ppm): 148.71 (OCOO), 130.46 (CH=CH), 72.22 (CH-CH₂-OCO), 32.55 (CH₂-CH=CH), 31.32 (CH-CH₂-OCO), 29.25-26.68 (CH₂). IR (cm⁻¹): 2918, 2850, 1726. Tₘ=59°C (melting temperature).

### Step 4B - Thiol-ene reaction: 6-membered cyclic biscarbonate synthesis (UndS-b6CC)

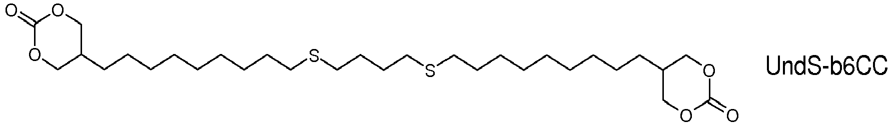

The Und-6CC obtained following Step 3 (4 g, 17.7 mmol) and 1,4-butanedithiol (2.38 g, 19.4 mmol, 1.1 eq.) were weighed into a flask. The reaction mixture was then UV-irradiated (254 nm) at room temperature. The reaction was monitored with ¹H NMR spectroscopy with the disappearance of the double bond. After completion of the reaction, ***UndS-b6CC*** was purified by recrystallization in a mixture of cyclohexane and ethyl acetate (70:30) and obtained as a white solid. Yield=37%. ¹H NMR (CDCl₃, 25°C, 400 MHz) δ (ppm): 4.40 (m, 4H), 4.09 (m, 4H), 2.52 (m, 8H), 2.20 (m, 2H), 1.68 (m, 4H), 1.57 (m, 4H), 1.35-1.28 (m, 28H). ¹³C NMR (CDCl₃, 25°C, 100 MHz) δ (ppm): 148.71 (OCOO), 72.24 (CH-CH₂-OCO), 32.31 and 31.90 (CH₂-S-CH₂), 31.42 (CH-CH₂-OCO), 29.78 and 26.72 (CH₂-CH₂-S-CH₂-CH₂), 29.60-26.72 (CH₂). IR (cm⁻¹): 2922, 2850, 1750, 1727. Tₘ=82°C (melting temperature).

### Example 2 - Kinetic experiments monitored by ¹H NMR

In order to identify precisely the difference in reactivity between 6-membered and 5-membered cyclic carbonates (activating or not) kinetic experiments have been performed. The monitoring of the kinetic of the reaction was carried out directly in NMR apparatus at the selected temperature. For the different substrates, the conversions were calculated from the decrease of the peaks corresponding to the cyclic carbonates. Then, based on the resulting kinetic curves, the orders of the reactions were determined together with the reaction rate constants.

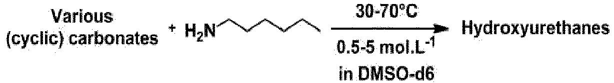

Various cyclic carbonate substrates such as trimethylene carbonate, Und-6CC, ethylene carbonate, vinyl ethylene carbonate, glycerol carbonate and propylene carbonate have been investigated on the reaction with hexylamine at 50°C and 1 mol.L⁻¹ in DMSO-d6, using TCB as a reference. Otherwise mentioned, the ratio between cyclic carbonate and amine was 1:1. With the ¹H NMR spectra at the initial times, a ratio of 1:1.0 to 1:1.18 was confirmed.

The kinetic experiments were performed in NMR tube at 50°C and 1 mol.L⁻¹ in DMSO-d6 and with a ratio 1:1 between cyclic carbonate and hexylamine. All reagents were dried before the reaction: on CaH₂ for hexylamine and on molecular sieves otherwise. For instance; a pre-solution of trimethyl carbonate (153 mg, 1.5 mmol, 3 eq.) and DMSO-d6 1.5 mL) is first prepared. Then, one third of this solution and trichlorobenzene as internal reference (12.5 µL, 0.1 mmol, 0.2 eq.) were added into an NMR tube. The hexylamine (66 µL, 0.5 mmol, 1 eq.) was then added just before putting the tube in the NRM apparatus. The reaction mixture was then heated at the reaction temperature. The reaction was monitored with ¹H NMR spectroscopy with the disappearance of the cyclic carbonate protons. After 7 hours or 15 hours, if the reaction was not completed, the NMR tube was placed in an oil bath at the desired temperature and reanalyzed later. For some experiments, the concentration (0.5 mol.L⁻¹ and 5 mol.L⁻¹) and the temperature (30°C and 70°C) were modulated. ¹H NMR, ¹³C NMR and IR detail are given below only for the reaction between Und-6CC+hexylamine.

The reaction rate constants kₐₚₚ for the reaction were calculated according to the ¹H NMR values.

The results are given in the following table:

| Cyclic carbonate | Concentration (mol.L⁻¹) | T (°C) | kₐₚₚ (L.mol⁻¹.h⁻¹) |
|---|---|---|---|
| Und-6CC | 1 | 30 | 0.46 |
| | 1 | 50 | 0.93 |
| | 1 | 70 | 1.92 |
| | 0.2 | 50 | 0.15 |
| | 5 | 50 | 1.77 |
| Trimethylene carbonate | 1 | 50 | 1.41 |
| | 1 | 50 | 1.86 |
| | 1 | 50 | 1.39 |
| Ethylene carbonate | 1 | 50 | 0.56 |
| Vinyl ethylene carbonate | 1 | 50 | 0.37 |
| Glycerol carbonate | 1 | 50 | 0.17 |
| Propylene carbonate | 1 | 50 | 0.08 |

***Product of the reaction between Und-6CC+hexylamine:*** ¹H NMR (DMSO-d6, 70°C, 400 MHz) δ (ppm): 6.71 (NH), 5.79 (m, 1 H), 4.97 (m, 2H), 3.94 (m, 2H), 3.41 (m, 2H), 2.98 (m, 2H), 2.02 (m, 2H), 1.66 (m, 1 H), 1.29 (m, 20H), 0.88 (t, 3H). ¹³C NMR (DMSO-d6, 70°C, 100 MHz) δ (ppm): 148.72 (OCOO), 139.14 (CH=CH₂), 114.62 (CH=CH₂), 64.62 (CH-CH₂-OCONH), 61.80 (CH-CH₂-OH), 41.83 (OH-CH₂-CH-CH₂-OCONH), 40.8 (CH₂-NHCOO), 33.50 (CH₂-CH=CH₂), 33.65-22.40 (CH₂), 13.94 (CH₃). IR (cm⁻¹): 3384, 2924, 2858, 1705.

### Example 3 - Polymer syntheses and characterizations

### General procedure for poly(hydroxyurethane)s

Poly(hydroxyurethane)s were prepared from the 6-membered cyclic biscarbonates (UndC20-b6CC and UndS-b6CC) and 1,12-diaminododecane (12DA). Poly(hydroxyurethane) syntheses were performed in DMF at room temperature or at 50°C into a schlenk tube under magnetic stirring and nitrogen atmosphere for 2 days. TCB was used as a reference. No catalysts were added for the polymerization reactions. Prior to MALDI-TOF MS analyses, the aliquots were quenched with hexylamine.

***PHU-2[UndC20-b6CC+12DA]:*** ¹H NMR (TCE-d2, 25°C, 400 MHz) δ (ppm): 5.39 (s, 2H), 4.86 (s, 2NH), 4.17-4.02 (m, 4H), 3.52-3.45 (m, 4H), 3.13 (m, 4H), 1.96 (m, 4H), 1.71 (m, 2H), 1.48-1.27 (m, CH₂). IR (cm⁻¹): 3620-3158, 2925, 2852, 1686, 1534, 1466, 1266, 1244, 1027, 962.

***PHU-3 [UndS-b6CC+12DA]:*** ¹H NMR (TCE-d2, 25°C, 400 MHz) δ (ppm): 4.89 (s, 2.NH), 4.23-4.09 (m, 4H), 3.58-3.49 (m, 4H), 3.17 (m, 4H), 2.57 (m, 8H), 1.77-1.72 (m, 6H), 1.62 (m, 4H), 1.51-1.33 (m, CH₂). IR (cm⁻¹): 3620-3158, 2925, 2852, 1686, 1534, 1466, 1266, 1244, 1027.

Thermal stability of poly(hydroxyurethane)s was investigated by TGA (Thermal Gravimetric Analysis) under a nitrogen stream at a heating rate of 10°C.min⁻¹. Degradation temperatures after 5 wt% loss were obtained up to 313°C.

The poly(hydroxyurethane)s were further analyzed by DSC (Differential Scanning Calorimetry) experiments.

The poly(hydroxyurethane)s from UndC20-b6CC were amorphous and present glass transition temperatures around -0°C.

The poly(hydroxyurethane) from UndS-b6CC was amorphous as well with a glass transition temperature of -20°C. This lower T_{g} can be explained by the presence of the sulfur atom, which is known to impart flexibility to the polymer chains.

Thus, the poly(hydroxyurethane)s obtained from the biscarbonates of the invention are thermoplastic polymers.

## Claims

1. A compound of formula (I): 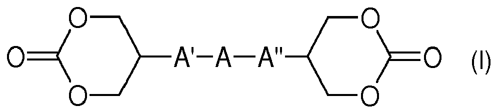 wherein:
- A is selected from the group consisting of -CH=CH- and -CH₂CHR'-S-R-S-CHR"CH₂-, wherein R is a linear or branched alkylene radical in C₂-C₆, preferably in C₄, optionally interrupted by one or more oxygen atoms, and wherein R' and R", identical or different, are selected from the group consisting of hydrogen atom and linear alkyl radicals in C₁-C₁₀; and
- A' and A", identical or different, are linear or branched alkylene radicals in C₁-C₁₂, preferably in C₆-C₁₂,
on the proviso that, when A is -CH₂CH₂-S-R-S-CH₂CH₂-, A' and A" are in C₂-C₁₂.

2. The compound according to claim 1, wherein the chain -A'-A-A"-comprises from 4 to 30 carbon atoms, preferably from 12 to 30 carbon atoms, advantageously from 16 to 24 carbon atoms.

3. The compound according to any one of claim 1 or 2, wherein:
- A is -CH=CH-, and
- A' and A" are linear alkylene radicals in C₆-C₁₂, preferably in C₇.

4. The compound according to any one of claim 1 or 2, wherein:
- A is -CH₂CH₂-S-R-S-CH₂CH₂-, R being as defined in claim 1, and
- A' and A" are linear alkylene radicals in C₆-C₁₂, preferably in C₇.

5. A compound having one of the following formulae:

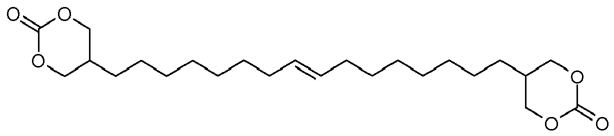

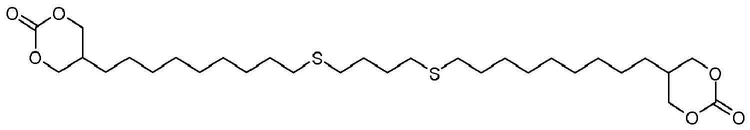

6. A method for preparing a compound of formula (I) according to any one of claims 1 to 3 wherein A is -CH=CH-, comprising a coupling step by metathesis reaction between a compound of formula (II-1): 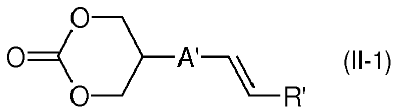 and a compound of formula (II-2): 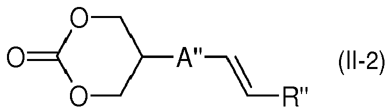 wherein A' and A" are as defined in any one of claims 1 to 3, and R' and R" are as defined in claim 1;
said step being carried out in a solvent in the presence of a metathesis catalyst, preferably selected from the Grubbs' catalysts.

7. A method for preparing a compound of formula (I) according to any one of claims 1, 2 and 4, wherein A is -CH₂CHR'-S-R-S-CHR"CH₂-, comprising a coupling step by thiol-ene reaction between a compound of formula (II-1): 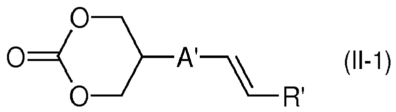 and a compound of formula (II-2): 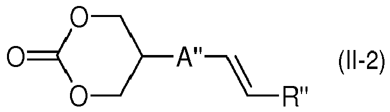 in the presence of a compound of formula HS-R-SH, preferably used as a solvent, wherein A' and A" are as defined in any one of claims 1, 2 and 4, and R, R' and R" are as defined in claim 1.

8. The method according to any one of claim 6 or 7, wherein the compound of formula (II-1) and the compound of formula (II-2) are prepared by a cyclization step of a compound of formula (III): 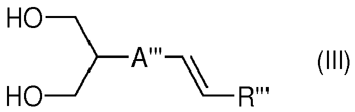 in the presence of a base and a compound of formula X-C(O)-O-R₁,
wherein:
- A"' represents either A' or A", A' and A" being as defined in any one of claims 1 to 4,
- R"' represents either R' or R", R' and R" being as defined in claim 1,
- X is OR₂, Br or Cl, preferably Cl, and
- R₁ and R₂, identical or different, are linear alkyl radicals in C₁-C₆, preferably in C₁-C₂.

9. The method according to claim 8, wherein the compound of formula (III) is prepared by a reduction step of a compound of formula (IV): 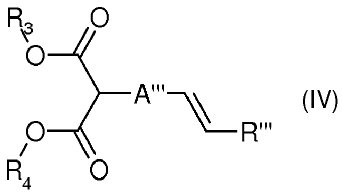 wherein:
- A"' and R"' are as defined in claim 8, and
- R₃ and R₄, identical or different, are linear alkyl radicals in C₁-C₆, preferably in C₁,
this step being conducted in a solvent in the presence of a metal hydride, such as LiAlH₄, or under pressurized H₂ in the presence of a catalyst, such as palladium over charcoal.

10. The method according to claim 9, wherein the compound of formula (IV) is prepared by a reaction step between a compound of formula (V): 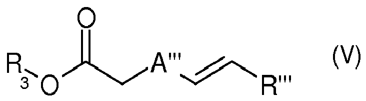 and a compound of formula R₄O-C(O)-OR₄,
wherein:
- A"' and R"' are as defined in claim 8, and
- R₃ and R₄ are as defined in claim 9,
this step being conducted in the presence of dimethylformamide and a metal hydride, such as NaH.

11. The use of a compound of formula (I) according to any one of claims 1 to 5, for the preparation of poly(hydroxyurethane)s or polycarbonates.

12. Polymer susceptible to be obtained by polymerization of a compound of formula (I) according to any one of claims 1 to 5 and at least one polyamine, said polyamine being in particular a diamine of formula H₂N-B-NH₂, wherein B is a linear alkylene radical in C₂-C₂₄, preferably in C₆-C₁₈, for example in C₁₂.

13. Polymer according to claim 12, said polymer consisting in n repetitive units of formula (U-1): 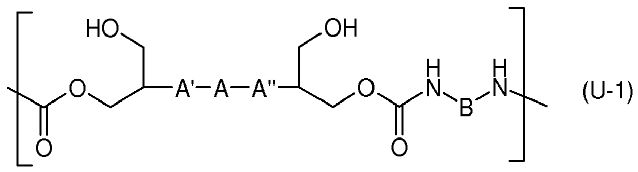 wherein n is comprised from 2 to 200, preferably from 10 to 200, A is as defined in claim 1, and A' and A" are as defined in any one of claims 1 to 4.

14. Synthesis intermediates having one of the following formulae: 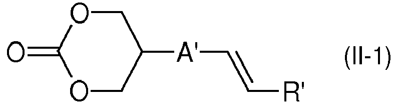 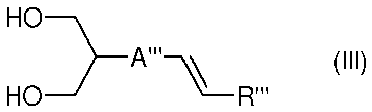 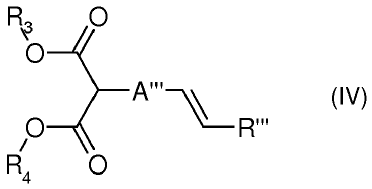 wherein formula (II-1) is as defined in claim 7, formula (III) is as defined in claim 8 and formula (IV) is as defined in claim 9.

15. Polymer susceptible to be obtained by homopolymerization of a compound of formula (II-1) according to claim 14, said polymer preferably consisting in n repetitive units of formula (U-2): 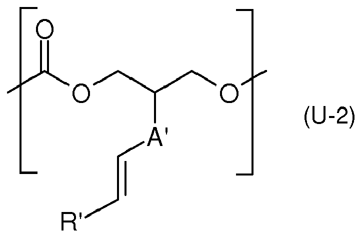 wherein n is comprised from 2 to 200, preferably from 10 to 200, A' and R' are as defined in claim 14.
